# EUROPÄISCHE PATENTANMELDUNG

(11) **EP 1 980 556 A1**
(43) Veröffentlichungstag der Anmeldung: **15.10.2008**
(21) Anmeldenummer: 07007493.5
(22) Anmeldetag: 12.04.2007
(51) Int. Cl.: C07D 231/20, A01N 43/56

(54) **4-(4-Trifluormethyl-3-thiobenzoyl)pyrazole und ihre Verwendung als Herbizide**

(71) Anmelder: Bayer CropScience AG, 40789 Monheim (DE)
(72) Erfinder: Ahrens, Hartmut, Dr., 63329 Egelsbach (DE); van Almsick, Andreas, Dr., 61184 Karben (DE); Schmitt, Monika, Dr., 60318 Frankfurt a.M. (DE); Dittgen, Jan, Dr., 60316 Frankfurt a.M. (DE); Hills, Martin, 65510 Idstein (DE); Kehne, Heinz, Dr., 65719 Hofheim (DE); Rosinger, Christopher, Dr., 65719 Hofheim (DE); Lehr, Stefan, Dr., 65835 Liederbach (DE); Feucht, Dieter, Dr., 65760 Eschborn (DE)

(57) **Zusammenfassung**

Es werden 4-(4-Trifluormethyl-3-thiobenzoyl)pyrazole der allgemeinen Formel (I) und ihre Verwendung als Herbizide beschrieben.

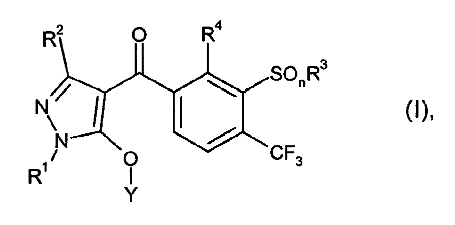

In dieser Formel (I) stehen R¹, R², R³ und R⁴ für Reste wie Wasserstoff und organische Reste wie Alkyl. Y bedeutet Wasserstoff oder eine Schutzgruppe wie Tosyl.

## Beschreibung

Die Erfindung betrifft das technische Gebiet der Herbizide, insbesondere das der Herbizide zur selektiven Bekämpfung von Unkräutern und Ungräsem in Nutzpflanzenkulturen.

Aus verschiedenen Schriften ist bereits bekannt, daß bestimmte Benzoylpyrazole herbizide Eigenschaften besitzen. So werden in DE 2513750, EP 0 352 543, EP 0 203 428, WO 97/41106, WO 00/03993 und US 4,557,753 Benzoylpyrazole genannt, die durch verschiedene Reste substituiert sind.

Die aus diesen Schriften bekannten Verbindungen zeigen jedoch häufig eine nicht ausreichende herbizide Wirksamkeit. Aufgabe der vorliegenden Erfindung ist daher die Bereitstellung von herbizid wirksamen Verbindungen mit - gegenüber den aus dem Stand der Technik bekannten Verbindungen - verbesserten herbiziden Eigenschaften.

Es wurde nun gefunden, daß bestimmte 4-Benzoylpyrazole, deren Phenylring in 3-Position eine Thiogruppe und in 4-Position eine Trifluormethylgruppe trägt, als Herbizide besonders gut geeignet sind. Ein Gegenstand der vorliegenden Erfindung sind 4-(4-Trifluormethyl-3-thiobenzoyl)pyrazole der Formel (I) oder deren Salze 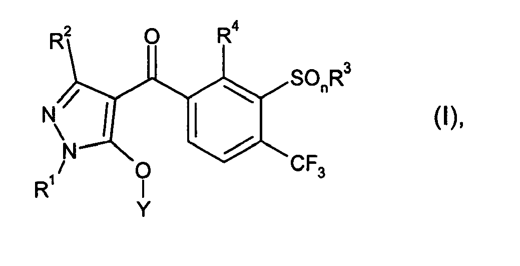 worin
- R¹: bedeutet (C₁-C₄)-Alkyl,
- R²: bedeutet Wasserstoff oder (C₁-C₄)-Alkyl,
- R³: bedeutet (C₁-C₄)-Alkyl,
- R⁴: bedeutet (C₁-C₄)-Alkyl,
- Y: bedeutet Wasserstoff, (C₁-C₆)-Alkylsulfonyl, (C₁-C₄)-Alkoxy-(C₁-C₆)-alkylsulfonyl, oder jeweils durch m gleiche oder verschiedene Reste aus der Gruppe bestehend aus Halogen, (C₁-C₄)-Alkyl und (C₁-C₄)-Alkoxy substituiertes Phenylsulfonyl, Thiophenyl-2-sulfonyl, Benzoyl, Benzoyl-(C₁-C₆)-alkyl oder Benzyl,
- m: bedeutet 0,1, 2 oder 3,
- n: bedeutet 0, 1 oder 2.

Für den Fall, daß Y Wasserstoff bedeutet, können die erfindungsgemäßen Verbindungen der Formel (I) in Abhängigkeit von äußeren Bedingungen, wie Lösungsmittel und pH-Wert, in unterschiedlichen tautomeren Strukturen auftreten:

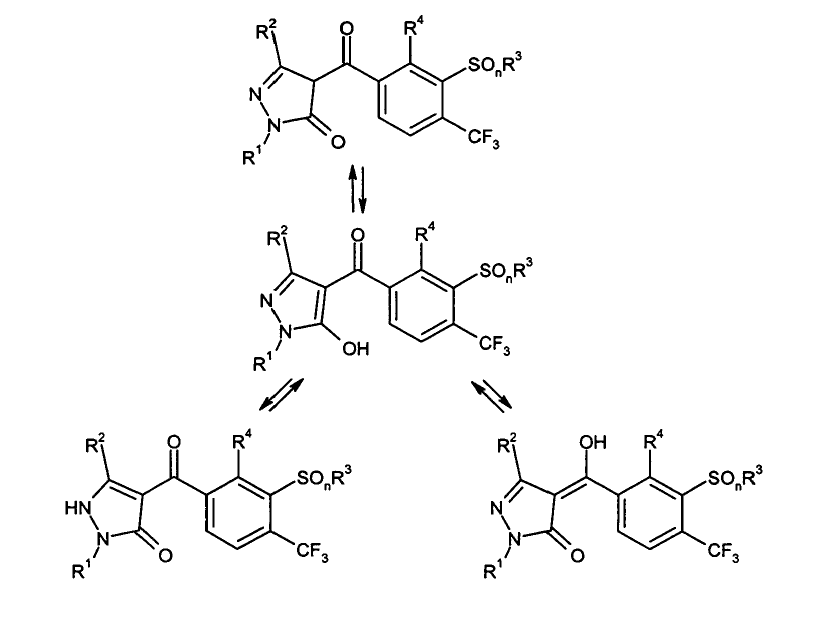

Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Lithium, Natrium, Kalium, Magnesium und Calcium sowie Ammoniak und organische Amine wie Triethylamin und Pyridin. Ebenso können Salze gebildet werden durch Anlagerung von organischen Säuren, wie Ameisen- oder Essigsäure, und anorganischen Säuren, wie Phosphor-, Salz- oder Schwefelsäure. Solche Salze sind ebenfalls Gegenstand der Erfindung.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder Iod. Tosyl bedeutet 4-Methylphenylsulfonyl.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Bevorzugt sind Verbindungen der allgemeinen Formel (I), worin
- R¹: bedeutet Methyl oder Ethyl,
- R²: bedeutet Wasserstoff, Methyl oder Ethyl,
- R³: bedeutet Methyl oder Ethyl,
- R⁴: bedeutet Methyl oder Ethyl,
- Y: bedeutet Wasserstoff, (C₁-C₃)-Alkylsulfonyl, (C₁-C₂)-Alkoxy-(C₁-C₄)-alkylsulfonyl, oder jeweils durch m Methylgruppen substituiertes Phenylsulfonyl, Thiophenyl-2-sulfonyl, Benzoyl, Benzoyl-(C₁-C₆)-alkyl oder Benzyl,
- m: bedeutet 0 oder 1,
- n: bedeutet 0, 1 oder 2.

Besonders bevorzugt sind Verbindungen der allgemeinen Formel (I), worin
- R¹: bedeutet Methyl oder Ethyl,
- R²: bedeutet Wasserstoff, Methyl oder Ethyl,
- R³: bedeutet Methyl oder Ethyl,
- R⁴: bedeutet Methyl oder Ethyl,
- Y: bedeutet Wasserstoff,
- n: bedeutet 0, 1 oder 2.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen Y für Wasserstoff steht, können beispielsweise nach dem in Schema 1 angegebenen und aus WO 98/42678 bekannten Verfahren durch basenkatalysierte Umsetzung eines Benzoesäurehalogenids (III) mit einem Pyrazolon (II) oder gemäß dem in Schema 2 angegebenen und beispielsweise aus EP-A 0 186 117 bekannten Verfahren durch basenkatalysierte Umsetzung eines Benzoesäurehalogenids (II) mit einem Pyrazolon (III) und anschließender Umlagerung hergestellt werden.

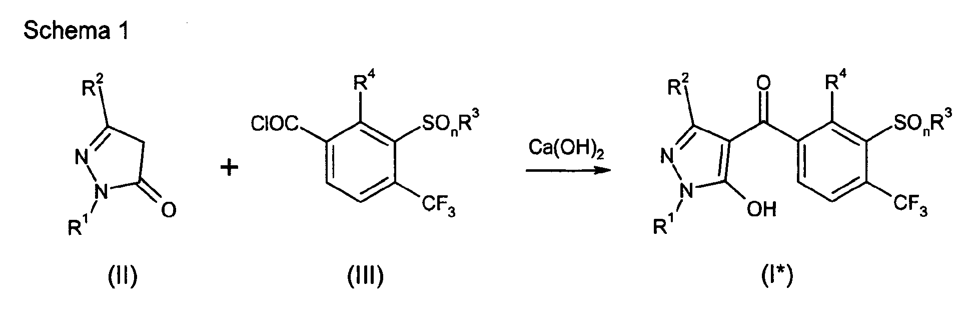

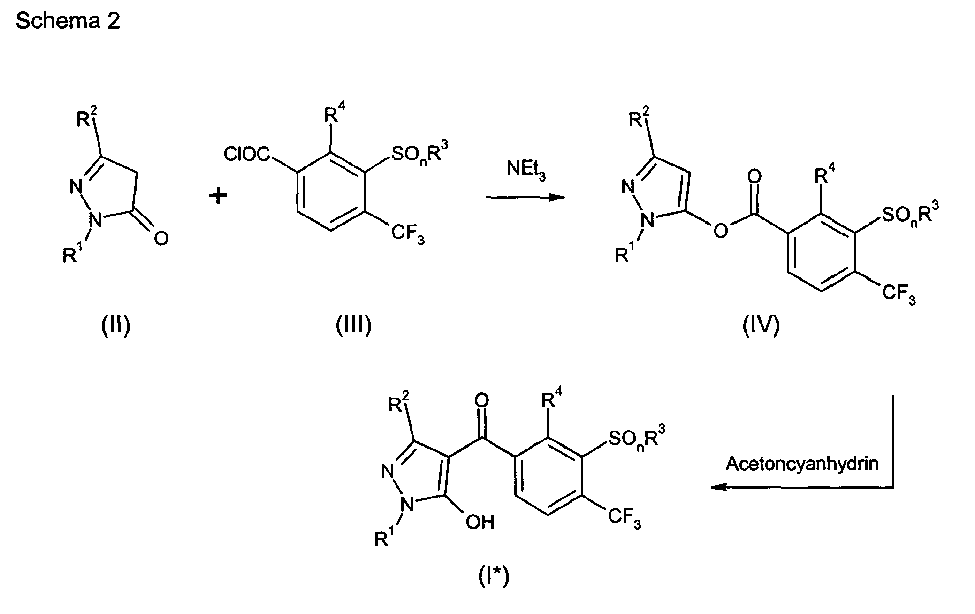

Erfindungsgemäße Verbindungen, in denen Y eine andere Bedeutung als Wasserstoff hat, werden gemäß Schema 3 zweckmäßigerweise aus den nach Schema 1 oder 2 erhältlichen Verbindungen durch basenkatalysierte Reaktion mit einem geeigneten Acylierungsmittel Y-X der Formel (V), worin X für eine Abgangsgruppe wie Halogen steht, hergestellt. Solche Methoden sind dem Fachmann grundsätzlich bekannt und beispielsweise in DOS 25 13 750 beschrieben.

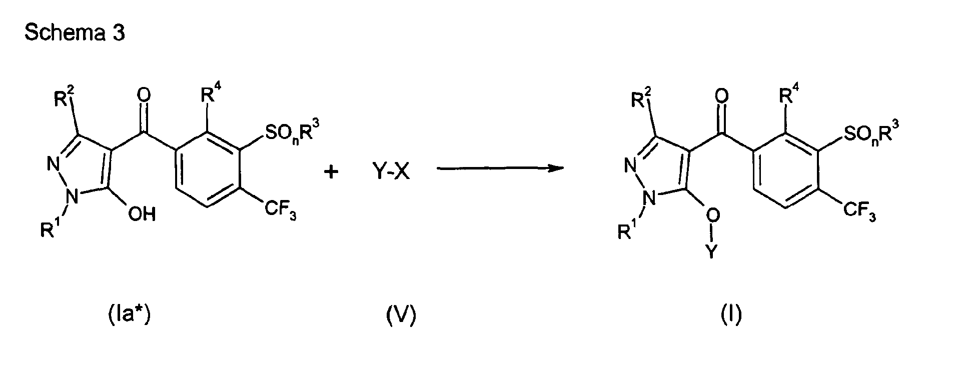

Erfindungsgemäße Verbindungen können auch gemäß dem in Schema 4 angegebenen und aus WO 98/42678 bekannten Verfahren durch Umsetzung eines Pyrazolons (II) mit einer Halogen-Benzoesäure (IIIa), anschließender nucleophiler aromatischer Substitution durch eine Thioverbindung HS-R³ und gegebenenfalls Oxidation der Thiogruppe hergestellt werden. Darin bedeutet L beispielsweise Chlor, Brom, Iod oder Trifluormethylsulfonyl. Solche Substitutionsreaktionen sind dem Fachmann bekannt und beispielsweise in Houben-Weyl, Methoden der Organischen Chemie, Georg Thieme Verlag Stuttgart, Bd. E 11, Erweiterungs- und Folgebände zur vierten Auflage 1985, S. 174 ff. beschrieben.

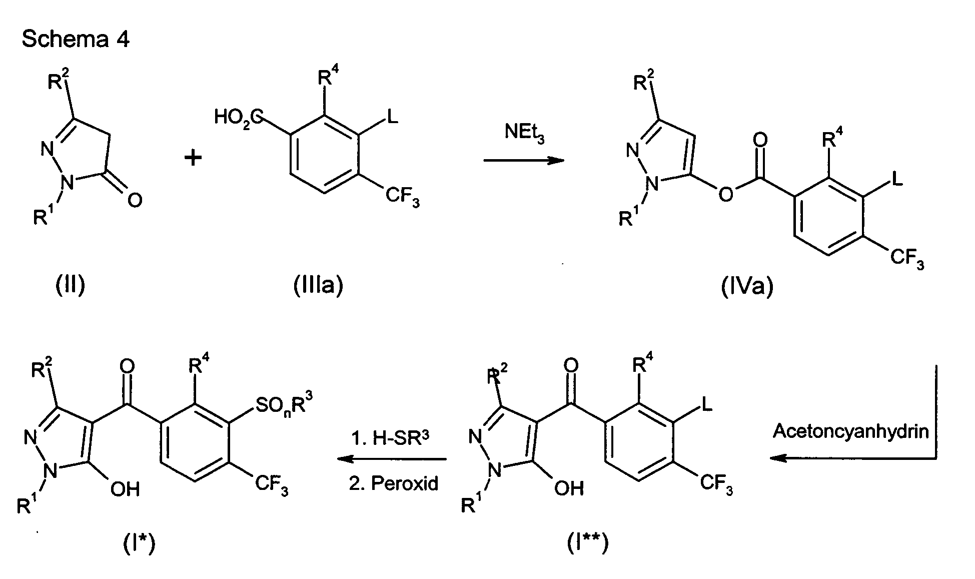

Die oben genannten Verbindungen der Formel (III) können beispielsweise durch Umsetzung mit Säurechloriden aus den Verbindungen der Formel (IIIb) gemäß dem Fachmann bekannten Methoden hergestellt werden.

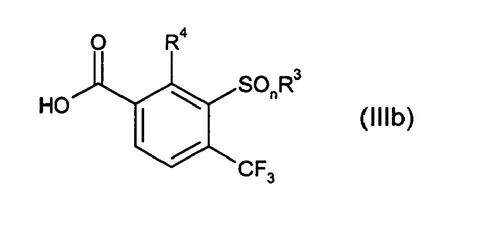

Verbindungen der Formel (III) und (IIIb), worin R³, R⁴ und n wie für Formel (I) definiert sind, sind neu und ebenfalls Gegenstand der vorliegenden Anmeldung.

Die in obigen Schemata verwendeten Ausgangsverbindungen sind entweder käuflich oder nach an sich bekannten Methoden herstellbar. So können die Pyrazolone der Formel (II) beispielsweise nach den in EP-A 0 240 001 und J. Prakt. Chem. 315, 382, (1973) beschriebenen Methoden und die Benzoylchloride der Formel (III) nach den in EP-A 0 527 036, WO 98/42678 und in JP 11292849 beschriebenen Verfahren hergestellt werden.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Apera spica venti, Chenopodium album, Lamium purpureum, Polygonum convulvulus, Stellaria media, Veronica hederifolia, Veronica persica und Viola tricolor.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Getreide, wie Weizen, Gerste und Mais, insbesondere Weizen, auf.
Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzen-kulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z.B. WO 92/11376, WO 92/14827, WO 91/19806),
- transgene Kulturpflanzen, welche gegen bestimmte Herbizide vom Typ Glufosinate (vgl. z.B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen. So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie ÖI-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetz-bar, wie sie z.B. in Weed Research 26, 441-445 (1986) oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone; CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl; chlorbromuron; chlorbufam; chlorfenac; chlorflurecol-methyl; chloridazon; chlorimuron ethyl; chlomitrofen, chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1 H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flucarbazone; flufenacet; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; foramsulfuron; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; iodosulfuron-methyl-natrium; isocarbamid; isopropalin; isoproturon; isouron; isoxaben; isoxapyrifop; karbutilate; lactofen; lenacil; linuron; MCPA; MCPB; mecoprop; mefenacet; mefluidid; mesosulfuron; mesotrione; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; pinoxaden; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propoxycarbazone; propyzamide; prosulfalin; prosulfocarb; prosulfuron (CGA-152005); prynachlor; pyrazolinate; pyrazon; pyrasulfotole; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulcotrione; sulfentrazon (FMC-97285, F-6285); sulfazuron; sulfometuron-methyl; sulfosate (ICI-A0224); TCA; tebutam (GCP-5544); tebuthiuron; tembotrione; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiencarbazone; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Herstellung von 1-Ethyl-4-(3'-ethylsulfonyl-2'-methyl-4'-trifluormethyl)benzoyl-5-hydroxypyrazol

### Schritt 1: 3-Fluor-2-methyl-4-trifluormethylbenzoesäure

25.0 g (120.1 mmol) 3-Fluor-4-trifluormethylbenzoesäure wurden in 250 ml THF gelöst, und 100.9 ml (2.5M in Hexan, 252.3 mmol) n-Butyllithium wurden bei -40 °C tropfenweise zugegeben. Das Gemisch wurde 3.5 h gerührt, anschließend wurde eine Lösung von 51.2 g (360.4 mmol) lodmethan in 50 ml trockenem THF tropfenweise zugegeben. Das Gemisch wurde 16 h gerührt, wobei nach einer halben Stunde die Temperatur langsam auf Raumtemperatur (RT) anstieg. Zur Aufarbeitung wurden vorsichtig 150 ml 1M HCl zugegeben. Das Gemisch wurde mit Diethylether extrahiert, anschließend wurde die organische Phase mit 1 M NaOH extrahiert. Die wäßrige Phase wurde angesäuert und danach mit Diethylether extrahiert. Die organische Phase wurde mit Wasser gewaschen, getrocknet und vom Lösungsmittel befreit. Der Rückstand wurde mit n-Heptan verrührt, und der Feststoff wurde über eine Filtration abgetrennt. Es wurden 13.5 g des sauberen Produkts isoliert.

### Schritt 2: 3-Ethylthio-2-methyl-4-trifluormethylbenzoesäure

3.00 g (13.5 mmol) 3-Fluor-2-methyl-4-trifluormethylbenzoesäure wurden in 50 ml N,N-Dimethylformamid vorgelegt. 1.68 g (60 Gew.-% Reinheit, 41.9 mmol) NaH wurden portionsweise zugegeben. Gegen Beendigung der Gasabspaltung wurden 1.77 g (95 Gew.-% Reinheit, 27.0 mmol) Ethanthiol tropfenweise zugegeben. Das Gemisch wurde 2 h lang bei RT gerührt und danach 10 h lang auf 80°C erhitzt. Das Reaktionsgemisch wurde abgekühlt, zur Aufarbeitung auf Eiswasser gegossen und danach mit konzentrierter Salzsäure angesäuert. Das Produkt fiel aus und wurde über eine Filtration abgetrennt. Es wurden 3.7 g des sauberen Produkts isoliert.

### Schritt 3: Synthese von 1-Ethyl-4-(3'-ethylthio-2'-methyl-4'-trifluormethyl)benzoyl-5-hydroxypyrazol

300 mg (1.14 mmol) 3-Ethylthio-2-methyl-4-trifluormethylbenzoesäure wurden in 20 ml trockenem CH₂Cl₂ vorgelegt und mit 288 mg (2.27 mmol) Oxalsäuredichlorid versetzt. Das Gemisch wurde für 15 min. auf Rückfluss erhitzt, danach war keine Gasabspaltung mehr zu beobachten. Der Inhalt wurde auf RT abgekühlt und eingeengt. Das so erhaltene Säurechlorid wurde in 20 ml trockenem CH₂Cl₂ gelöst und die Lösung wurde zu einem Gemisch aus 140 mg (1.25 mmol) 1-Ethyl-5-hydroxypyrazol und katalytischen Mengen 4-N,N-Dimethylaminopyridin gegeben. Danach wurden 230 mg (2.27 mmol) Triethylamin langsam zugetropft, und das Reaktionsgemisch wurde 16 h bei RT gerührt. Zur Aufarbeitung wurden 3 ml 1 M HCl zugegeben und nach der Phasentrennung wurde die organische Phase vom Lösungsmittel befreit. Der so erhaltene Enolester wurde in 20 ml Acetonitril aufgenommen, und es wurden 230 mg (2.27 mmol) Triethylamin zugegeben. Anschließend wurden acht Tropfen Acetoncyanhydrin sowie eine Spatelspitze KCN zugegeben. Das Gemisch wurde 16 h bei RT gerührt und wurde dann eingeengt. Der Rückstand wurde mit 20 ml CH₂Cl₂ und anschließend mit 3 ml 1 M HCl versetzt. Nach der Phasentrennung wurde das Lösungsmittel abgetrennt. Der Rückstand wurde chromatographisch gereinigt, wobei 182 mg sauberes Produkt isoliert wurden.

### Schritt 4: Synthese von 1-Ethyl-4-(3'-ethylsulfonyl-2'-methyl-4'-trifluormethyl) benzoyl-5-hydroxypyrazol

182 mg (0.51 mmol) 1-Ethyl-4-(3'-ethylthio-2'-methyl-4'-trifluormethyl)benzoyl-5-hydroxypyrazol wurden in 20 ml CH₂Cl₂ gelöst und anschließend mit 376 mg (70 Gew.-% Reinheit, 1.52 mmol) Metachlorperbenzoesäure versetzt. Danach wurde das Gemisch 16 h bei RT gerührt. Zur Aufarbeitung wurde mit CH₂Cl₂ verdünnt und mit 10 %-iger wässriger Natriumhydrogensulfit-Lösung gewaschen. Es wurde mit 1 M HCl angesäuert, anschließend wurde nach der Phasentrennung und nach dem analytischen Nachweis der Abwesenheit von Peroxiden die organische Phase getrocknet und vom Lösungsmittel befreit. Der Rückstand wurde chromatographisch gereinigt, wobei 88.8 mg sauberes Produkt isoliert wurden.

### Herstellung von 5-Hydroxy-1,3-dimethyl-4-(2'-methyl-3'-methylsulfonyl-4'-trifluormethyl)benzoylpyrazol

### Schritt 1: Synthese von 2-Methyl-3-methylthio-4-trifluormethylbenzoesäure

300 mg (1.35 mmol) 3-Fluor-2-methyl-4-trifluormethylbenzoesäure wurden in 5 ml N,N-Dimethylformamid vorgelegt, und 59 mg (60 Gew.-% Reinheit, 1.49 mmol) NaH wurden zugegeben. Das Gemisch wurde 10 min. gerührt, anschließend wurden 199 mg (95 Gew.-% Reinheit, 2.70 mmol) Natriumthiomethylat zugegeben. Das Gemisch wurde 1.5 h lang bei RT gerührt und danach 16 h lang auf 80 °C erhitzt. Das Reaktionsgemisch wurde abgekühlt, zur Aufarbeitung auf Wasser gegossen, mit Essigsäureethylester extrahiert, und danach wurde die wässrige Phase mit konzentrierter HCl angesäuert. Das Gemisch wurde zweimal mit t-Butylmethylether extrahiert, getrocknet und eingeengt. Es wurden 310 mg des Produkts isoliert.

### Schritt 2: Synthese von 2-Methyl-3-methylsulfonyl-4-trifluormethylbenzoesäure

1.50 g (5.99 mmol) 2-Methyl-3-methylthio-4-trifluormethylbenzoesäure wurden in 20 ml Eisessig vorgelegt. 59 mg (0.18 mmol) Natriumwolframat(VI)dihydrat wurden zugegeben, danach wurde das Gemisch auf 50- 60°C erhitzt. Bei dieser Temperatur wurden vorsichtig 2.45 ml (30 %-ig, 23.98 mmol) einer wäßrigen Wasserstoffperoxid-Lösung tropfenweise zugegeben. Das Gemisch wurde einige Stunden bei dieser Temperatur gerührt, bis die HPLC-Analyse kein Edukt und kein Sulfoxid mehr anzeigte. Das Reaktionsgemisch wurde abgekühlt und zur Aufarbeitung auf Wasser gegossen. Das Gemisch wurde dreimal mit Essigsäureethylester extrahiert, die vereinigten organischen Phasen wurden mit einer wäßrigen gesättigten Natriumhydrogensulfit-Lösung gewaschen, und nach dem analytischen Nachweis der Abwesenheit von Peroxiden wurde das Gemisch mit 1 M HCl angesäuert. Die organische Phase wurde getrocknet und von den Lösungsmitteln befreit. Es wurden 1.67 g des sauberen Produkts isoliert.

### Schritt 3: Synthese von 5-Hydroxy-1,3-dimethyl-4-(2'-methyl-3'-methylsulfonyl-4'-trifluormethyl)benzoylpyrazol

200 mg (0.71 mmol) 2-Methyl-3-methylsulfonyl-4-trifluormethylbenzoesäure wurden zusammen mit 87 mg (0.78 mmol) 5-Hydroxy-1,3-dimethylpyrazol sowie einer katalytischen Menge 4-N,N-Dimethylaminopyridin in 20 ml trockenem CH₂Cl₂ vorgelegt und mit 163 mg (0.85 mmol) 1-(3-Dimethylaminopropyl)-3-ethylcarbodiimid Hydrochlorid versetzt. Das Gemisch wurde 3 h bei RT gerührt und dann mit 3 ml 1 M HCl versetzt. Nach der Phasentrennung wurde die wässrige Phase mit CH₂Cl₂ extrahiert. Die organischen Phasen wurden getrocknet und eingeengt. Der Rückstand wurde in 20 ml Acetonitril und 143 mg (1.42 mmol) Triethylamin aufgenommen und mit acht Tropfen Acetoncyanhydrin sowie einer Spatelspitze KCN versetzt. Das Gemisch wurde 16 h bei RT gerührt und wurde eingeengt. Der Rückstand wurde mit 15 ml CH₂Cl₂ und anschließend mit 2 ml 1 M HCl versetzt. Nach der Phasentrennung wurde die wässrige Phase mit CH₂Cl₂ extrahiert. Die vereinigten organischen Phasen wurden getrocknet und eingengt. Der Rückstand wurde chromatographisch gereinigt. Es wurden 112.7 mg sauberes Produkt isoliert.

Die in nachfolgenden Tabellen aufgeführten Beispiele wurden analog oben genannten Methoden hergestellt beziehungsweise sind analog oben genannten Methoden erhältlich. Diese Verbindungen sind besonders bevorzugt.

Die verwendeten Abkürzungen bedeuten:

| | | | |
|---|---|---|---|
| Bu = Butyl | Et = Ethyl | Me = Methyl | Pr = Propyl |
| i = iso | s = sekundär | t = tertiär | Ph = Phenyl |

**Tabelle A: Erfindungsgemäße Verbindungen der allgemeinen Formel (I), worin R¹ für Methyl steht, sowie R² und Y jeweils Wasserstoff bedeuten**

| | | | | |
|---|---|---|---|---|
| 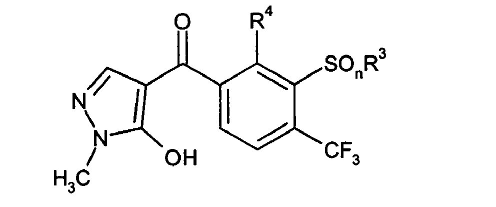 | | | | |

| **Nr.** | **R³** | **R⁴** | **n** | **Physikalische Daten:** ¹H-NMR: δ [CDCl₃] |
|---|---|---|---|---|
| 1-1 | Me | Me | 0 | 7.68 (d, 1 H), 7.45 (d, 1 H), 7.30 (s, 1 H), 3.71 (s, 3H), 2.68 (s, 3H), 2.31 (s, 3H) |
| 1-2 | Et | Me | 0 | 7.69 (d, 1 H), 7.44 (d, 1 H), 7.31 (s, 1 H), 3.72 (s, 3H), 2.77 (q, 2H), 2.66 (s, 3H), 1.22 (t, 3H) |
| 1-3 | n-Pr | Me | 0 | |
| 1-4 | i-Pr | Me | 0 | |
| 1-5 | n-Bu | Me | 0 | |
| 1-6 | i-Bu | Me | 0 | |
| 1-7 | s-Bu | Me | 0 | |
| 1-8 | t-Bu | Me | 0 | |
| 1-9 | Me | Me | 1 | 7.71 (d, 1 H), 7.56 (d, 1 H), 7.28 (s, 1 H), 3.72 (s, 3H), 3.03 (s, 3H), 2.90 (s, 3H) |
| 1-10 | Et | Me | 1 | 7.71 (d, 1 H), 7.56 (d, 1H), 7.26 (s, 1 H), 3.72 (s, 3H), 3.47 (dq, 1 H), 2.96 (dq, 1 H), 2.85 (s, 3H), 1.43 (t, 3H) |
| 1-11 | n-Pr | Me | 1 | |
| 1-12 | i-Pr | Me | 1 | |
| 1-13 | n-Bu | Me | 1 | |
| 1-14 | i-Bu | Me | 1 | |
| 1-15 | s-Bu | Me | 1 | |
| 1-16 | t-Bu | Me | 1 | |
| 1-17 | Me | Me | 2 | 7.91 (d, 1 H), 7.67 (d, 1 H), 7.27 (s, 1 H), 3.72 (s, 3H), 3.27 (s, 3H), 2.76 (s, 3H) |
| 1-18 | Et | Me | 2 | |
| 1-19 | n-Pr | Me | 2 | |
| 1-20 | i-Pr | Me | 2 | |
| 1-21 | n-Bu | Me | 2 | |
| 1-22 | i-Bu | Me | 2 | |
| 1-23 | s-Bu | Me | 2 | |
| 1-24 | t-Bu | Me | 2 | |
| 1-25 | Me | Et | 0 | 7.68 (d, 1 H), 7.42 (d, 1 H), 7.30 (s, 1 H), 3.71 (s, 3H), 3.16 (q, 2H), 2.36 (s, 3H), 1.19 (t, 3H) |
| 1-26 | Et | Et | 0 | |
| 1-27 | n-Pr | Et | 0 | |
| 1-28 | i-Pr | Et | 0 | |
| 1-29 | n-Bu | Et | 0 | |
| 1-30 | i-Bu | Et | 0 | |
| 1-31 | s-Bu | Et | 0 | |
| 1-32 | t-Bu | Et | 0 | |
| 1-33 | Me | Et | 1 | |
| 1-34 | Et | Et | 1 | |
| 1-35 | n-Pr | Et | 1 | |
| 1-36 | i-Pr | Et | 1 | |
| 1-37 | n-Bu | Et | 1 | |
| 1-38 | i-Bu | Et | 1 | |
| 1-39 | s-Bu | Et | 1 | |
| 1-40 | t-Bu | Et | 1 | |
| 1-41 | Me | Et | 2 | |
| 1-42 | Et | Et | 2 | |
| 1-43 | n-Pr | Et | 2 | |
| 1-44 | i-Pr | Et | 2 | |
| 1-45 | n-Bu | Et | 2 | |
| 1-46 | i-Bu | Et | 2 | |
| 1-47 | s-Bu | Et | 2 | |
| 1-48 | t-Bu | Et | 2 | |

**Tabelle B: Erfindungsgemäße Verbindungen der allgemeinen Formel (I), worin R¹ für Ethyl steht, sowie R² und Y jeweils Wasserstoff bedeuten**

| | | | | |
|---|---|---|---|---|
| 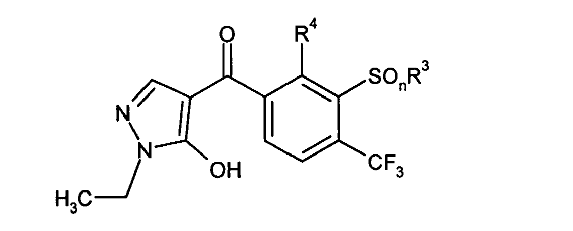 | | | | |

| **Nr.** | **R³** | **R⁴** | **n** | **Physikalische Daten:** ¹H-NMR: δ [CDCl₃] |
|---|---|---|---|---|
| 2-1 | Me | Me | 0 | 7.69 (d, 1 H), 7.47 (d, 1 H), 7.31 (s, 1 H), 4.08 (q, 2H), 2.69 (s, 3H), 2.31 (s, 3H), 1.46 (t, 3H) |
| 2-2 | Et | Me | 0 | 7.68 (d, 1 H), 7.46 (d, 1 H), 7.31 (s, 1 H), 4.07 (q, 2H), 2.76 (q, 2H), 2.66 (s, 3H), 1.45 (t, 3H), 1.22 (t, 3H) |
| 2-3 | n-Pr | Me | 0 | |
| 2-4 | i-Pr | Me | 0 | |
| 2-5 | n-Bu | Me | 0 | |
| 2-6 | i-Bu | Me | 0 | |
| 2-7 | s-Bu | Me | 0 | |
| 2-8 | t-Bu | Me | 0 | |
| 2-9 | Me | Me | 1 | 7.71 (d, 1 H), 7.57 (d, 1 H), 7.29 (s, 1 H), 4.09 (q, 2H), 3.03 (s, 3H), 2.90 (s, 3H), 1.46 (t, 3H) |
| 2-10 | Et | Me | 1 | 7.72 (d, 1 H), 7.57 (d, 1 H), 7.27 (s, 1 H), 4.08 (q, 2H), 3.47 (dq, 1 H), 2.96 (dq, 1 H), 2.87 (s, 3H), 1.49 -1.41 (m, 6H) |
| 2-11 | n-Pr | Me | 1 | |
| 2-12 | i-Pr | Me | 1 | |
| 2-13 | n-Bu | Me | 1 | |
| 2-14 | i-Bu | Me | 1 | |
| 2-15 | s-Bu | Me | 1 | |
| 2-16 | t-Bu | Me | 1 | |
| 2-17 | Me | Me | 2 | 7.91 (d, 1 H), 7.67 (d, 1 H), 7.28 (s, 1 H), 4.08 (q, 2H), 3.27 (s, 3H), 2.78 (s, 3H), 1.46 (t, 3H) |
| 2-18 | Et | Me | 2 | 7.91 (d, 1 H), 7.65 (d, 1 H), 7.27 (s, 1 H), 4.10 (q, 2H), 3.36 (q, 2H), 2.77 (s, 3H), 1.51 - 1.42 (m, 6H) |
| 2-19 | n-Pr | Me | 2 | |
| 2-20 | i-Pr | Me | 2 | |
| 2-21 | n-Bu | Me | 2 | |
| 2-22 | i-Bu | Me | 2 | |
| 2-23 | s-Bu | Me | 2 | |
| 2-24 | t-Bu | Me | 2 | |
| 2-25 | Me | Et | 0 | 7.69 (d, 1H), 7.45 (d, 1H), 7.30 (s, 1 H), 4.08 (q, 2H), 3.16 (q, 2H), 2.36 (s, 3H), 1.46 (t, 3H), 1.20 (t, 3H) |
| 2-26 | Et | Et | 0 | |
| 2-27 | n-Pr | Et | 0 | |
| 2-28 | i-Pr | Et | 0 | |
| 2-29 | n-Bu | Et | 0 | |
| 2-30 | i-Bu | Et | 0 | |
| 2-31 | s-Bu | Et | 0 | |
| 2-32 | t-Bu | Et | 0 | |
| 2-33 | Me | Et | 1 | |
| 2-34 | Et | Et | 1 | |
| 2-35 | n-Pr | Et | 1 | |
| 2-36 | i-Pr | Et | 1 | |
| 2-37 | n-Bu | Et | 1 | |
| 2-38 | i-Bu | Et | 1 | |
| 2-39 | s-Bu | Et | 1 | |
| 2-40 | t-Bu | Et | 1 | |
| 2-41 | Me | Et | 2 | 7.90 (d, 1H), 7.65 (d, 1 H), 7.28 (s, 1 H), 4.10 (q, 2H), 3.32 (q, 2H), 3.29 (s, 3H), 1.46 (t, 3H), 1.23 (t, 3H) |
| 2-42 | Et | Et | 2 | |
| 2-43 | n-Pr | Et | 2 | |
| 2-44 | i-Pr | Et | 2 | |
| 2-45 | n-Bu | Et | 2 | |
| 2-46 | i-Bu | Et | 2 | |
| 2-47 | s-Bu | Et | 2 | |
| 2-48 | t-Bu | Et | 2 | |

**Tabelle C: Erfindungsgemäße Verbindungen der allgemeinen Formel (I), worin R¹ und R² jeweils Methyl bedeuten, sowie Y für Wasserstoff steht**

| | | | | |
|---|---|---|---|---|
| 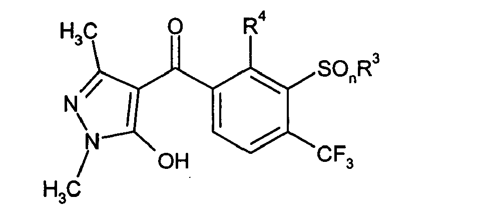 | | | | |

| **Nr.** | **R³** | **R⁴** | **n** | **Physikalische Daten:** ¹H-NMR: δ [CDCl₃] |
|---|---|---|---|---|
| 3-1 | Me | Me | 0 | 7.69 (d, 1 H), 7.29 (d, 1 H), 3.62 (s, 3H), 2.60 (s, 3H), 2.29 (s, 3H), 1.62 (s, 3H) |
| 3-2 | Et | Me | 0 | 7.72 (d, 1 H), 7.30 (d, 1 H), 3.66 (s, 3H), 2.78 (q, 2H), 2.59 (s, 3H), 1.66 (s, 3H), 1.26 (t, 3H) |
| 3-3 | n-Pr | Me | 0 | |
| 3-4 | i-Pr | Me | 0 | |
| 3-5 | n-Bu | Me | 0 | |
| 3-6 | i-Bu | Me | 0 | |
| 3-7 | s-Bu | Me | 0 | |
| 3-8 | t-Bu | Me | 0 | |
| 3-9 | Me | Me | 1 | 7.72 (d, 1 H), 7.42 (d, 1 H), 3.66 (s, 3H), 3.01 (s, 3H), 2.82 (s, 3H), 1.69 (s, 3H) |
| 3-10 | Et | Me | 1 | 400 MHz-1 H-NMR (CDCl3): 7.72 (d, 1 H), 7.42 (d, 1 H), 3.65 (s, 3H), 3.44 (dq, 1 H), 2.93 (dq, 1 H), 2.78 (s, 3H), 1.67 (s, 3H), 1.42 (t, 3H) |
| 3-11 | n-Pr | Me | 1 | |
| 3-12 | i-Pr | Me | 1 | |
| 3-13 | n-Bu | Me | 1 | |
| 3-14 | i-Bu | Me | 1 | |
| 3-15 | s-Bu | Me | 1 | |
| 3-16 | t-Bu | Me | 1 | |
| 3-17 | Me | Me | 2 | 7.91 (d, 1 H), 7.53 (d, 1 H), 3.65 (s, 3H), 3.23 (s, 3H), 2.71 (s, 3H), 1.68 (s, 3H) |
| 3-18 | Et | Me | 2 | |
| 3-19 | n-Pr | Me | 2 | |
| 3-20 | i-Pr | Me | 2 | |
| 3-21 | n-Bu | Me | 2 | |
| 3-22 | i-Bu | Me | 2 | |
| 3-23 | s-Bu | Me | 2 | |
| 3-24 | t-Bu | Me | 2 | |
| 3-25 | Me | Et | 0 | 7.70 (d, 1H), 7.28 (d, 1 H), 3.63 (s, 3H), 3.42 (q, 2H), 2.32 (s, 3H), 1.63 (s, 3H), 1.18 (t, 3H) |
| 3-26 | Et | Et | 0 | |
| 3-27 | n-Pr | Et | 0 | |
| 3-28 | i-Pr | Et | 0 | |
| 3-29 | n-Bu | Et | 0 | |
| 3-30 | i-Bu | Et | 0 | |
| 3-31 | s-Bu | Et | 0 | |
| 3-32 | t-Bu | Et | 0 | |
| 3-33 | Me | Et | 1 | |
| 3-34 | Et | Et | 1 | |
| 3-35 | n-Pr | Et | 1 | |
| 3-36 | i-Pr | Et | 1 | |
| 3-37 | n-Bu | Et | 1 | |
| 3-38 | i-Bu | Et | 1 | |
| 3-39 | s-Bu | Et | 1 | |
| 3-40 | t-Bu | Et | 1 | |
| 3-41 | Me | Et | 2 | |
| 3-42 | Et | Et | 2 | |
| 3-43 | n-Pr | Et | 2 | |
| 3-44 | i-Pr | Et | 2 | |
| 3-45 | n-Bu | Et | 2 | |
| 3-46 | i-Bu | Et | 2 | |
| 3-47 | s-Bu | Et | 2 | |
| 3-48 | t-Bu | Et | 2 | |

**Tabelle D: Erfindungsgemäße Verbindungen der allgemeinen Formel (I)**

| | | | | | | | |
|---|---|---|---|---|---|---|---|
| 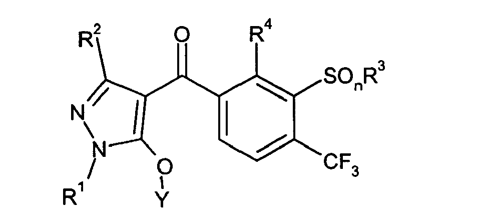 | | | | | | | |

| **Nr.** | **R¹** | **R²** | **R³** | **R⁴** | **n** | **Y** | **Physikalische Daten:** ¹H-NMR: δ [CDCl₃] |
|---|---|---|---|---|---|---|---|
| 4-1 | Me | H | Me | Me | 0 | - SO₂-n-Pr | |
| 4-2 | Me | H | Et | Me | 0 | - SO₂-n-Pr | |
| 4-3 | Me | H | n-Pr | Me | 0 | - SO₂-n-Pr | |
| 4-4 | Me | H | Me | Me | 1 | - SO₂-n-Pr | |
| 4-5 | Me | H | Et | Me | 1 | - SO₂-n-Pr | |
| 4-6 | Me | H | n-Pr | Me | 1 | - SO₂-n-Pr | |
| 4-7 | Me | H | Me | Me | 2 | - SO₂-n-Pr | |
| 4-8 | Me | H | Et | Me | 2 | -SO₂-n-Pr | |
| 4-9 | Me | H | n-Pr | Me | 2 | -SO₂-n-Pr | |
| 4-10 | Me | H | Me | Me | 0 | -SO₂-(CH₂)₂OMe | |
| 4-11 | Me | H | Et | Me | 0 | - SO₂-(CH₂)₂OMe | |
| 4-12 | Me | H | n-Pr | Me | 0 | - SO₂-(CH₂)₂OMe | |
| 4-13 | Me | H | Me | Me | 1 | - SO₂-(CH₂)₂OMe | |
| 4-14 | Me | H | Et | Me | 1 | - SO₂-(CH₂)₂OMe | |
| 4-15 | Me | H | n-Pr | Me | 1 | - SO₂-(CH₂)₂OMe | |
| 4-16 | Me | H | Me | Me | 2 | - SO₂-(CH₂)₂OMe | |
| 4-17 | Me | H | Et | Me | 2 | - SO₂-(CH₂)₂OMe | |
| 4-18 | Me | H | n-Pr | Me | 2 | - SO₂-(CH₂)₂OMe | |
| 4-19 | Me | H | Me | Me | 0 | -SO₂-Ph | |
| 4-20 | Me | H | Et | Me | 0 | -SO₂-Ph | |
| 4-21 | Me | H | n-Pr | Me | 0 | -SO₂-Ph | |
| 4-22 | Me | H | Me | Me | 1 | -SO₂-Ph | |
| 4-23 | Me | H | Et | Me | 1 | -SO₂-Ph | |
| 4-24 | Me | H | n-Pr | Me | 1 | -SO₂-Ph | |
| 4-25 | Me | H | Me | Me | 2 | -SO₂-Ph | |
| 4-26 | Me | H | Et | Me | 2 | -SO₂-Ph | |
| 4-27 | Me | H | n-Pr | Me | 2 | - SO₂-Ph | |
| 4-28 | Me | H | Me | Me | 0 | - SO₂-(4-Me-Ph) | |
| 4-29 | Me | H | Et | Me | 0 | - SO₂-(4-Me-Ph) | |
| 4-30 | Me | H | n-Pr | Me | 0 | - SO₂-(4-Me-Ph) | |
| 4-31 | Me | H | Me | Me | 1 | - SO₂-(4-Me-Ph) | |
| 4-32 | Me | H | Et | Me | 1 | - SO₂-(4-Me-Ph) | |
| 4-33 | Me | H | n-Pr | Me | 1 | - SO₂-(4-Me-Ph) | |
| 4-34 | Me | H | Me | Me | 2 | - SO₂-(4-Me-Ph) | |
| 4-35 | Me | H | Et | Me | 2 | - SO₂-(4-Me-Ph) | |
| 4-36 | Me | H | n-Pr | Me | 2 | - SO₂-(4-Me-Ph) | |
| 4-37 | Me | H | Me | Me | 0 | - SO₂-(Thien-2-yl) | |
| 4-38 | Me | H | Et | Me | 0 | - SO₂-(Thien-2-yl) | |
| 4-39 | Me | H | n-Pr | Me | 0 | - SO₂-(Thien-2-yl) | |
| 4-40 | Me | H | Me | Me | 1 | - SO₂-(Thien-2-yl) | |
| 4-41 | Me | H | Et | Me | 1 | - SO₂-(Thien-2-yl) | |
| 4-42 | Me | H | n-Pr | Me | 1 | - SO₂-(Thien-2-yl) | |
| 4-43 | Me | H | Me | Me | 2 | - SO₂-(Thien-2-yl) | |
| 4-44 | Me | H | Et | Me | 2 | - SO₂-(Thien-2-yl) | |
| 4-45 | Me | H | n-Pr | Me | 2 | - SO₂-(Thien-2-yl) | |
| 4-46 | Me | H | Me | Me | 0 | - CO-Ph | |
| 4-47 | Me | H | Et | Me | 0 | - CO-Ph | |
| 4-48 | Me | H | n-Pr | Me | 0 | -CO-Ph | |
| 4-49 | Me | H | Me | Me | 1 | -CO-Ph | |
| 4-50 | Me | H | Et | Me | 1 | -CO-Ph | |
| 4-51 | Me | H | n-Pr | Me | 1 | -CO-Ph | |
| 4-52 | Me | H | Me | Me | 2 | -CO-Ph | |
| 4-53 | Me | H | Et | Me | 2 | -CO-Ph | |
| 4-54 | Me | H | n-Pr | Me | 2 | -CO-Ph | |
| 4-55 | Me | H | Me | Me | 0 | -CH₂-CO-Ph | |
| 4-56 | Me | H | Et | Me | 0 | -CH₂-CO-Ph | |
| 4-57 | Me | H | n-Pr | Me | 0 | - CH₂-CO-Ph | |
| 4-58 | Me | H | Me | Me | 1 | - CH₂-CO-Ph | |
| 4-59 | Me | H | Et | Me | 1 | - CH₂-CO-Ph | |
| 4-60 | Me | H | n-Pr | Me | 1 | - CH₂-CO-Ph | |
| 4-61 | Me | H | Me | Me | 2 | - CH₂-CO-Ph | |
| 4-62 | Me | H | Et | Me | 2 | - CH₂-CO-Ph | |
| 4-63 | Me | H | n-Pr | Me | 2 | - CH₂-CO-Ph | |
| 4-64 | Et | H | Me | Me | 0 | - SO₂-n-Pr | |
| 4-65 | Et | H | Et | Me | 0 | - SO₂-n-Pr | |
| 4-66 | Et | H | n-Pr | Me | 0 | - SO₂-n-Pr | |
| 4-67 | Et | H | Me | Me | 1 | - SO₂-n-Pr | |
| 4-68 | Et | H | Et | Me | 1 | - SO₂-n-Pr | |
| 4-69 | Et | H | n-Pr | Me | 1 | - SO₂-n-Pr | |
| 4-70 | Et | H | Me | Me | 2 | - SO₂-n-Pr | |
| 4-71 | Et | H | Et | Me | 2 | - SO₂-n-Pr | |
| 4-72 | Et | H | n-Pr | Me | 2 | - SO₂-n-Pr | |
| 4-73 | Et | H | Me | Me | 0 | - SO₂-(CH₂)₂OMe | |
| 4-74 | Et | H | Et | Me | 0 | - SO₂-(CH₂)₂OMe | |
| 4-75 | Et | H | n-Pr | Me | 0 | - SO₂-(CH₂)₂OMe | |
| 4-76 | Et | H | Me | Me | 1 | - SO₂-(CH₂)₂OMe | |
| 4-77 | Et | H | Et | Me | 1 | - SO₂-(CH₂)₂OMe | |
| 4-78 | Et | H | n-Pr | Me | 1 | - SO₂-(CH₂)₂OMe | |
| 4-79 | Et | H | Me | Me | 2 | - SO₂-(CH₂)₂OMe | |
| 4-80 | Et | H | Et | Me | 2 | - SO₂-(CH₂)₂OMe | |
| 4-81 | Et | H | n-Pr | Me | 2 | - SO₂-(CH₂)₂OMe | |
| 4-82 | Et | H | Me | Me | 0 | - SO₂-Ph | |
| 4-83 | Et | H | Et | Me | 0 | - SO₂-Ph | |
| 4-84 | Et | H | n-Pr | Me | 0 | - SO₂-Ph | |
| 4-85 | Et | H | Me | Me | 1 | - SO₂-Ph | |
| 4-86 | Et | H | Et | Me | 1 | - SO₂-Ph | |
| 4-87 | Et | H | n-Pr | Me | 1 | - SO₂-Ph | |
| 4-88 | Et | H | Me | Me | 2 | - SO₂-Ph | |
| 4-89 | Et | H | Et | Me | 2 | - SO₂-Ph | |
| 4-90 | Et | H | n-Pr | Me | 2 | - SO₂-Ph | |
| 4-91 | Et | H | Me | Me | 0 | - SO₂-(4-Me-Ph) | |
| 4-92 | Et | H | Et | Me | 0 | - SO₂-(4-Me-Ph) | |
| 4-93 | Et | H | n-Pr | Me | 0 | - SO₂-(4-Me-Ph) | |
| 4-94 | Et | H | Me | Me | 1 | - SO₂-(4-Me-Ph) | |
| 4-95 | Et | H | Et | Me | 1 | - SO₂-(4-Me-Ph) | |
| 4-96 | Et | H | n-Pr | Me | 1 | - SO₂-(4-Me-Ph) | |
| 4-97 | Et | H | Me | Me | 2 | - SO₂-(4-Me-Ph) | |
| 4-98 | Et | H | Et | Me | 2 | - SO₂-(4-Me-Ph) | |
| 4-99 | Et | H | n-Pr | Me | 2 | - SO₂-(4-Me-Ph) | |
| 4-100 | Et | H | Me | Me | 0 | - SO₂-(Thien-2-yl) | |
| 4-101 | Et | H | Et | Me | 0 | - SO₂-(Thien-2-yl) | |
| 4-102 | Et | H | n-Pr | Me | 0 | - SO₂-(Thien-2-yl) | |
| 4-103 | Et | H | Me | Me | 1 | - SO₂-(Thien-2-yl) | |
| 4-104 | Et | H | Et | Me | 1 | -SO₂-(Thien-2-yl) | |
| 4-105 | Et | H | n-Pr | Me | 1 | - SO₂-(Thien-2-yl) | |
| 4-106 | Et | H | Me | Me | 2 | - SO₂-(Thien-2-yl) | |
| 4-107 | Et | H | Et | Me | 2 | - SO₂-(Thien-2-yl) | |
| 4-108 | Et | H | n-Pr | Me | 2 | - SO₂-(Thien-2-yl) | |
| 4-109 | Et | H | Me | Me | 0 | -CO-Ph | |
| 4-110 | Et | H | Et | Me | 0 | -CO-Ph | |
| 4-111 | Et | H | n-Pr | Me | 0 | -CO-Ph | |
| 4-112 | Et | H | Me | Me | 1 | -CO-Ph | |
| 4-113 | Et | H | Et | Me | 1 | - CO-Ph | |
| 4-114 | Et | H | n-Pr | Me | 1 | -CO-Ph | |
| 4-115 | Et | H | Me | Me | 2 | -CO-Ph | |
| 4-116 | Et | H | Et | Me | 2 | -CO-Ph | |
| 4-117 | Et | H | n-Pr | Me | 2 | -CO-Ph | |
| 4-118 | Et | H | Me | Me | 0 | -CH₂-CO-Ph | |
| 4-119 | Et | H | Et | Me | 0 | - CH₂-CO-Ph | |
| 4-120 | Et | H | n-Pr | Me | 0 | -CH₂-CO-Ph | |
| 4-121 | Et | H | Me | Me | 1 | -CH₂-CO-Ph | |
| 4-122 | Et | H | Et | Me | 1 | -CH₂-CO-Ph | |
| 4-123 | Et | H | n-Pr | Me | 1 | - CH₂-CO-Ph | |
| 4-124 | Et | H | Me | Me | 2 | - CH₂-CO-Ph | |
| 4-125 | Et | H | Et | Me | 2 | - CH₂-CO-Ph | |
| 4-126 | Et | H | n-Pr | Me | 2 | - CH₂-CO-Ph | |
| 4-127 | Me | Me | Me | Me | 0 | - SO₂-n-Pr | |
| 4-128 | Me | Me | Et | Me | 0 | - SO₂-n-Pr | |
| 4-129 | Me | Me | n-Pr | Me | 0 | - SO₂-n-Pr | |
| 4-130 | Me | Me | Me | Me | 1 | - SO₂-n-Pr | |
| 4-131 | Me | Me | Et | Me | 1 | - SO₂-n-Pr | |
| 4-132 | Me | Me | n-Pr | Me | 1 | - SO₂-n-Pr | |
| 4-133 | Me | Me | Me | Me | 2 | - SO₂-n-Pr | |
| 4-134 | Me | Me | Et | Me | 2 | - SO₂-n-Pr | |
| 4-135 | Me | Me | n-Pr | Me | 2 | - SO₂-n-Pr | |
| 4-136 | Me | Me | Me | Me | 0 | - SO₂-(CH₂)₂OMe | |
| 4-137 | Me | Me | Et | Me | 0 | - SO₂-(CH₂)₂OMe | |
| 4-138 | Me | Me | n-Pr | Me | 0 | - SO₂-(CH₂)₂OMe | |
| 4-139 | Me | Me | Me | Me | 1 | - SO₂-(CH₂)₂OMe | |
| 4-140 | Me | Me | Et | Me | 1 | - SO₂-(CH₂)₂OMe | |
| 4-141 | Me | Me | n-Pr | Me | 1 | - SO₂-(CH₂)₂OMe | |
| 4-142 | Me | Me | Me | Me | 2 | - SO₂-(CH₂)₂OMe | |
| 4-143 | Me | Me | Et | Me | 2 | - SO₂-(CH₂)₂OMe | |
| 4-144 | Me | Me | n-Pr | Me | 2 | - SO₂-(CH₂)₂OMe | |
| 4-145 | Me | Me | Me | Me | 0 | - SO₂-Ph | |
| 4-146 | Me | Me | Et | Me | 0 | - SO₂-Ph | |
| 4-147 | Me | Me | n-Pr | Me | 0 | - SO₂-Ph | |
| 4-148 | Me | Me | Me | Me | 1 | - SO₂-Ph | |
| 4-149 | Me | Me | Et | Me | 1 | - SO₂-Ph | |
| 4-150 | Me | Me | n-Pr | Me | 1 | - SO₂-Ph | |
| 4-151 | Me | Me | Me | Me | 2 | - SO₂-Ph | |
| 4-152 | Me | Me | Et | Me | 2 | - SO₂-Ph | |
| 4-153 | Me | Me | n-Pr | Me | 2 | - SO₂-Ph | |
| 4-154 | Me | Me | Me | Me | 0 | - SO₂-(4-Me-Ph) | |
| 4-155 | Me | Me | Et | Me | 0 | - SO₂-(4-Me-Ph) | |
| 4-156 | Me | Me | n-Pr | Me | 0 | - SO₂-(4-Me-Ph) | |
| 4-157 | Me | Me | Me | Me | 1 | - SO₂-(4-Me-Ph) | |
| 4-158 | Me | Me | Et | Me | 1 | - SO₂-(4-Me-Ph) | |
| 4-159 | Me | Me | n-Pr | Me | 1 | -SO₂-(4-Me-Ph) | |
| 4-160 | Me | Me | Me | Me | 2 | -SO₂-(4-Me-Ph) | |
| 4-161 | Me | Me | Et | Me | 2 | - SO₂-(4-Me-Ph) | |
| 4-162 | Me | Me | n-Pr | Me | 2 | -SO₂-(4-Me-Ph) | |
| 4-163 | Me | Me | Me | Me | 0 | - SO₂-(Thien-2-yl) | |
| 4-164 | Me | Me | Et | Me | 0 | - SO₂-(Thien-2-yl) | |
| 4-165 | Me | Me | n-Pr | Me | 0 | - SO₂-(Thien-2-yl) | |
| 4-166 | Me | Me | Me | Me | 1 | - SO₂-(Thien-2-yl) | |
| 4-167 | Me | Me | Et | Me | 1 | - SO₂-(Thien-2-yl) | |
| 4-168 | Me | Me | n-Pr | Me | 1 | - SO₂-(Thien-2-yl) | |
| 4-169 | Me | Me | Me | Me | 2 | -SO₂-(Thien-2-yl) | |
| 4-170 | Me | Me | Et | Me | 2 | - SO₂-(Thien-2-yl) | |
| 4-171 | Me | Me | n-Pr | Me | 2 | - SO₂-(Thien-2-yl) | |
| 4-172 | Me | Me | Me | Me | 0 | -CO-Ph | |
| 4-173 | Me | Me | Et | Me | 0 | -CO-Ph | |
| 4-174 | Me | Me | n-Pr | Me | 0 | -CO-Ph | |
| 4-175 | Me | Me | Me | Me | 1 | -CO-Ph | |
| 4-176 | Me | Me | Et | Me | 1 | -CO-Ph | |
| 4-177 | Me | Me | n-Pr | Me | 1 | -CO-Ph | |
| 4-178 | Me | Me | Me | Me | 2 | -CO-Ph | |
| 4-179 | Me | Me | Et | Me | 2 | - CO-Ph | |
| 4-180 | Me | Me | n-Pr | Me | 2 | - CO-Ph | |
| 4-181 | Me | Me | Me | Me | 0 | - CH₂-CO-Ph | |
| 4-182 | Me | Me | Et | Me | 0 | - CH₂-CO-Ph | |
| 4-183 | Me | Me | n-Pr | Me | 0 | - CH₂-CO-Ph | |
| 4-184 | Me | Me | Me | Me | 1 | - CH₂-CO-Ph | |
| 4-185 | Me | Me | Et | Me | 1 | - CH₂-CO-Ph | |
| 4-186 | Me | Me | n-Pr | Me | 1 | - CH₂-CO-Ph | |
| 4-187 | Me | Me | Me | Me | 2 | - CH₂-CO-Ph | |
| 4-188 | Me | Me | Et | Me | 2 | - CH₂-CO-Ph | |
| 4-189 | Me | Me | n-Pr | Me | 2 | - CH₂-CO-Ph | |

**Tabelle E: Erfindungsgemäße Verbindungen der allgemeinen Formel (IIIb)**

| | | | | |
|---|---|---|---|---|
| 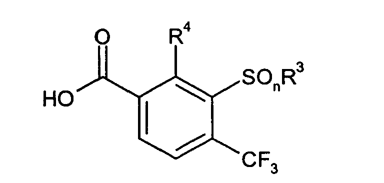 | | | | |

| **Nr.** | **R³** | **R⁴** | n | **Physikalische Daten:** ¹H-NMR: δ [CDCl₃] |
|---|---|---|---|---|
| 5-1 | Me | Me | 0 | 7.98 (d, 1 H), 7.66 (d, 1 H), 2.92 (s, 3H), 2.28 (s, 3H) |
| 5-2 | Et | Me | 0 | 400 MHz-¹H-NMR (CDCl₃): 7.98 (d, 1 H), 7.67 (d, 1 H), 2.91 (s, 3H), 2.75 (q, 2H), 1.22 (t, 3H) |
| 5-3 | n-Pr | Me | 0 | |
| 5-4 | i-Pr | Me | 0 | |
| 5-5 | n-Bu | Me | 0 | |
| 5-6 | i-Bu | Me | 0 | |
| 5-7 | s-Bu | Me | 0 | |
| 5-8 | t-Bu | Me | 0 | |
| 5-9 | Me | Me | 1 | (DMSO-d₆): 7.92 (d, 1 H), 7.81 (d, 1 H), 3.04 (s, 3H), 2.91 (s, 3H) |
| 5-10 | Et | Me | 1 | (DMSO-d₆): 7.92 (d, 1 H), 7.82 (d, 1 H), 3.46 (dq, 1 H), 3.02 (dq, 1 H), 2.88 (s, 3H), 1.27 (t, 3H) |
| 5-11 | n-Pr | Me | 1 | |
| 5-12 | i-Pr | Me | 1 | |
| 5-13 | n-Bu | Me | 1 | |
| 5-14 | i-Bu | Me | 1 | |
| 5-15 | s-Bu | Me | 1 | |
| 5-16 | t-Bu | Me | 1 | |
| 5-17 | Me | Me | 2 | 8.11 (d, 1H), 7.87 (d, 1H), 3.30 (s, 3H), 2.96 (s, 3H) |
| 5-18 | Et | Me | 2 | |
| 5-19 | n-Pr | Me | 2 | |
| 5-20 | i-Pr | Me | 2 | |
| 5-21 | n-Bu | Me | 2 | |
| 5-22 | i-Bu | Me | 2 | |
| 5-23 | s-Bu | Me | 2 | |
| 5-24 | t-Bu | Me | 2 | |
| 5-25 | Me | Et | 0 | |
| 5-26 | Et | Et | 0 | |
| 5-27 | n-Pr | Et | 0 | |
| 5-28 | i-Pr | Et | 0 | |
| 5-29 | n-Bu | Et | 0 | |
| 5-30 | i-Bu | Et | 0 | |
| 5-31 | s-Bu | Et | 0 | |
| 5-32 | t-Bu | Et | 0 | |
| 5-33 | Me | Et | 1 | |
| 5-34 | Et | Et | 1 | |
| 5-35 | n-Pr | Et | 1 | |
| 5-36 | i-Pr | Et | 1 | |
| 5-37 | n-Bu | Et | 1 | |
| 5-38 | i-Bu | Et | 1 | |
| 5-39 | s-Bu | Et | 1 | |
| 5-40 | t-Bu | Et | 1 | |
| 5-41 | Me | Et | 2 | 8.07 (d, 1 H), 7.87 (d, 1H), 3.50 (q, 2H), 3.29 (s, 3H), 1.36 (t, 3H) |
| 5-42 | Et | Et | 2 | |
| 5-43 | n-Pr | Et | 2 | |
| 5-44 | i-Pr | Et | 2 | |
| 5-45 | n-Bu | Et | 2 | |
| 5-46 | i-Bu | Et | 2 | |
| 5-47 | s-Bu | Et | 2 | |
| 5-48 | t-Bu | Et | 2 | |

**Tabelle F: Erfindungsgemäße Verbindungen der allgemeinen Formel (III)**

| | | | | |
|---|---|---|---|---|
| 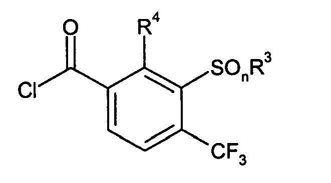 | | | | |

| **Nr.** | **R³** | **R⁴** | **n** | **Physikalische Daten:** ¹H-NMR: δ [CDCl₃] |
|---|---|---|---|---|
| 6-1 | Me | Me | 0 | |
| 6-2 | Et | Me | 0 | |
| 6-3 | n-Pr | Me | 0 | |
| 6-4 | i-Pr | Me | 0 | |
| 6-5 | n-Bu | Me | 0 | |
| 6-6 | i-Bu | Me | 0 | |
| 6-7 | s-Bu | Me | 0 | |
| 6-8 | t-Bu | Me | 0 | |
| 6-9 | Me | Me | 1 | |
| 6-10 | Et | Me | 1 | |
| 6-11 | n-Pr | Me | 1 | |
| 6-12 | i-Pr | Me | 1 | |
| 6-13 | n-Bu | Me | 1 | |
| 6-14 | i-Bu | Me | 1 | |
| 6-15 | s-Bu | Me | 1 | |
| 6-16 | t-Bu | Me | 1 | |
| 6-17 | Me | Me | 2 | |
| 6-18 | Et | Me | 2 | |
| 6-19 | n-Pr | Me | 2 | |
| 6-20 | i-Pr | Me | 2 | |
| 6-21 | n-Bu | Me | 2 | |
| 6-22 | i-Bu | Me | 2 | |
| 6-23 | s-Bu | Me | 2 | |
| 6-24 | t-Bu | Me | 2 | |
| 6-25 | Me | Et | 0 | |
| 6-26 | Et | Et | 0 | |
| 6-27 | n-Pr | Et | 0 | |
| 6-28 | i-Pr | Et | 0 | |
| 6-29 | n-Bu | Et | 0 | |
| 6-30 | i-Bu | Et | 0 | |
| 6-31 | s-Bu | Et | 0 | |
| 6-32 | t-Bu | Et | 0 | |
| 6-33 | Me | Et | 1 | |
| 6-34 | Et | Et | 1 | |
| 6-35 | n-Pr | Et | 1 | |
| 6-36 | i-Pr | Et | 1 | |
| 6-37 | n-Bu | Et | 1 | |
| 6-38 | i-Bu | Et | 1 | |
| 6-39 | s-Bu | Et | 1 | |
| 6-40 | t-Bu | Et | 1 | |
| 6-41 | Me | Et | 2 | |
| 6-42 | Et | Et | 2 | |
| 6-43 | n-Pr | Et | 2 | |
| 6-44 | i-Pr | Et | 2 | |
| 6-45 | n-Bu | Et | 2 | |
| 6-46 | i-Bu | Et | 2 | |
| 6-47 | s-Bu | Et | 2 | |
| 6-48 | t-Bu | Et | 2 | |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | | |
|---|---|---|
| 75 | Gew.-Teile einer Verbindung der allgemeinen Formel (I), | |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | | |
|---|---|---|
| 25 | Gew.-Teile einer Verbindung der allgemeinen Formel (I), | |
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 800l/ha in unterschiedlichen Dosierungen auf die Oberfläche der Abdeckerde appliziert. Anschließend werden die Töpfe zur weiteren Kultivierung der Pflanzen im Gewächshaus unter optimalen Bedingungen gehalten. Die optische Bewertung der Schäden an den Schadpflanzen erfolgt 3-4 Wochen nach der Behandlung. Wie die Ergebnisse dieser Vergleichstabellen zeigen, weisen die ausgewählten erfindungsgemäßen Verbindungen dabei eine besssere herbizide Wirksamkeit gegen ein breites Spektrum wirtschaftlich wichtiger mono- und dikotyler Schadpflanzen auf als die im Stand der Technik offenbarten

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 I/ha in einer in den Tabellen 1 bis 5 angegebenen Dosierung auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der erfindungsgemäßen Verbindungen im Vergleich zu Verbindungen, die im Stand der Technik offenbart sind, bonitiert. Wie die Ergebnisse dieser Vergleichstabellen zeigen, weisen die ausgewählten erfindungsgemäßen Verbindungen dabei eine besssere herbizide Wirksamkeit gegen ein breites Spektrum wirtschaftlich wichtiger mono- und dikotyler Schadpflanzen auf als die im Stand der Technik offenbarten.

Die in folgenden Vergleichstabellen verwendeten Abkürzungen bedeuten:

| | | | |
|---|---|---|---|
| ABUTH | Abutilon theophrasti | AMARE | Amaranthus retroflexus |
| AVEFA | Avena fatua | DIGSA | Digitaria sanguinalis |
| ECHCG | Echinochloa crus galli | GALAP | Galium aparine |
| LOLMU | Lolium multiflorum | MATIN | Matricaria inodora |
| POLCO | Polygonum convolvulus | SETVI | Setaria viridis |
| STEME | Stellaria media | VERPE | Veronica persica |
| VIOTR | Viola tricolor | XANST | Xanthium strumarium |
| StdT | Stand der Technik | | |

### Vergleichstabelle 1: Vorauflauf

| **Verbindung Nr.** | **Dosierung [ga.i./ha]** | **Wirkung gegen Schadpflanzen** | | |
|---|---|---|---|---|
| | | **ECHCG** | **LOLMU** | **ABUTH** |
| 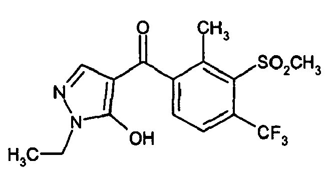 | 80 | 100% | 70% | 100% |
| erfindungsgemäße Verbindung | | | | |
| 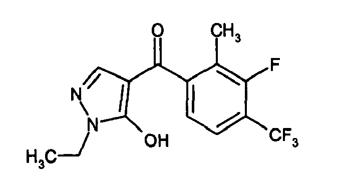 | 80 | 30% | 0% | 0% |
| aus dem StdT bekannte Verbindung | | | | |

### Vergleichstabelle 2: Vorauflauf

| **Verbindung Nr.** | **Dosierung [g a.i/.ha]** | **Wirkung gegen Schadpflanzen** | | |
|---|---|---|---|---|
| | | **AMARE** | **STEME** | **VERPE** |
| 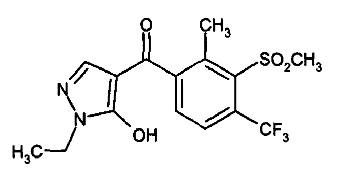 | 80 | 90% | 90% | 90% |
| erfindungsgemäße Verbindung | | | | |
| 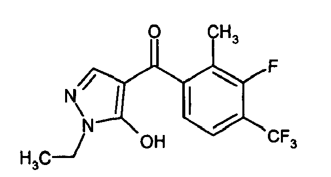 | 80 | 0% | 40% | 0% |
| aus dem StdT bekannte Verbindung | | | | |

### Vergleichstabelle 3: Nachauflauf

| **Verbindung Nr.** | **Dosierung [g a.i./ha]** | **Wirkung gegen Schadpflanzen** | | |
|---|---|---|---|---|
| | | **ECHCG** | **LOLMU** | **SETVI** |
| 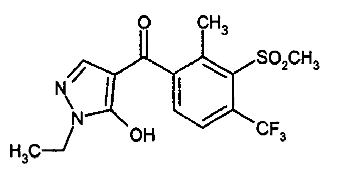 | 80 | 90% | 60% | 100% |
| erfindungsgemäße Verbindung | | | | |
| 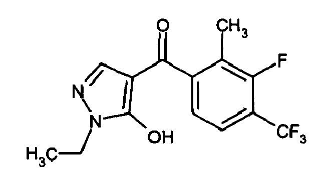 | 80 | 0% | 0% | 0% |
| aus dem StdT bekannte Verbindung | | | | |

### Vergleichstabelle 4: Nachauflauf

| **Verbindung Nr.** | **Dosierung [g a.i./ha]** | **Wirkung gegen Schadpflanzen** | | |
|---|---|---|---|---|
| | | **ABUTH** | **AMARE** | **MATIN** |
| 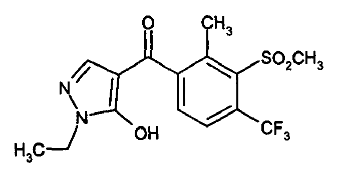 | 80 | 90% | 100% | 90% |
| erfindungsgemäße Verbindung | | | | |
| 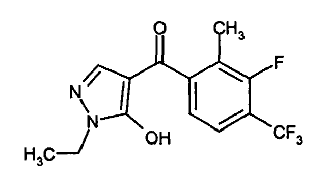 | 80 | 80% | 0% | 0% |
| aus dem StdT bekannte Verbindung | | | | |

### Vergleichstabelle 5: Nachauflauf

| **Verbindung Nr** | **Dosierung [g a.i./ha]** | **Wirkung gegen Schädpflänzen** | | | |
|---|---|---|---|---|---|
| | | **POLCO** | **STEME** | **VERPE** | **VIOTR** |
| 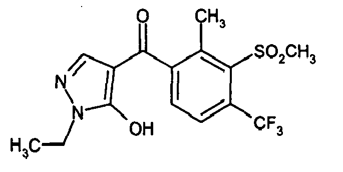 | 80 | 100% | 90% | 90% | 80% |
| erfindungsgemäße Verbindung | | | | | |
| 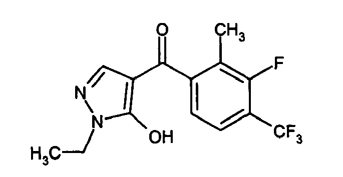 | 80 | 60% | 0% | 0% | 0% |
| aus dem StdT bekannte Verbindung | | | | | |

### Vergleichstabelle 6: Vorauflauf

| **Verbindung Nr.** | **Dosierung [g a.i./ha]** | **Wirkung gegen Schadpflanzen** | | | |
|---|---|---|---|---|---|
| | | **AVESA** | **LOLMU** | **SETVI** | **AMARE** |
| 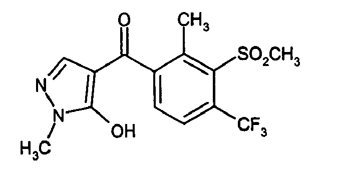 | 320 | 90% | 90% | 100% | 100% |
| erfindungsgemäße Verbindung | | | | | |
| 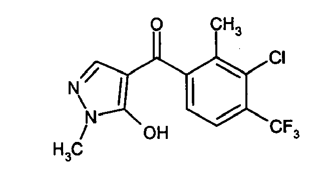 | 320 | 20% | 20% | 20% | 50% |
| aus dem StdT bekannte Verbindung | | | | | |

### Vergleichstabelle 7: Nachauflauf

| **Verbindung Nr.** | **Dosierung [g a.i./ha]** | **Wirkung gegen Schadpflanzen** | | | |
|---|---|---|---|---|---|
| | | **AVESA** | **ECHCG** | **LOLMU** | **SETVI** |
| 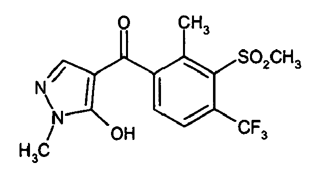 | 80 | 70% | 90% | 80% | 90% |
| erfindungsgemäße Verbindung | | | | | |
| 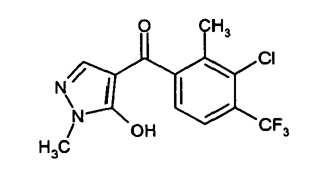 | 320 | 20% | 0% | 20% | 30% |
| aus dem StdT bekannte Verbindung | | | | | |

### Vergleichstabelle 8: Vorauflauf

| **Verbindung Nr.** | **Dosierung [g a.i./ha]** | **Wirkung gegen Schadpflanzen** | | |
|---|---|---|---|---|
| | | **LOLMU** | **GALAP** | **POLCO** |
| 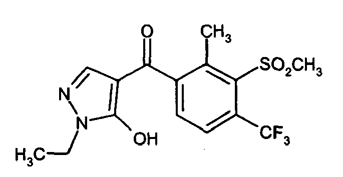 | 80 | 70% | 80% | 50% |
| erfindungsgemäße Verbindung | | | | |
| 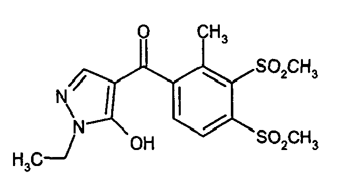 | 80 | 0% | 60% | 30% |
| aus dem StdT bekannte Verbindung | | | | |

### Vergleichstabelle 9: Vorauflauf

| **Verbindung Nr.** | **Dosierung [g a.i./ha]** | **Wirkung gegen Schadpflanzen** | | |
|---|---|---|---|---|
| | | **VERPE** | **VIOTR** | **XANST** |
| 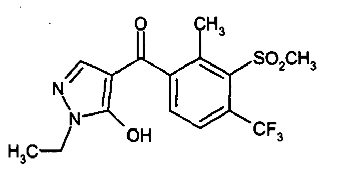 | 80 | 90% | 90% | 100% |
| erfindungsgemäße Verbindung | | | | |
| 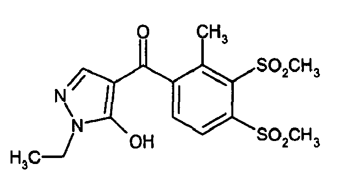 | 80 | 80% | 20% | 40% |
| aus dem StdT bekannte Verbindung | | | | |

### Vergleichstabelle 10: Nachauflauf

| **Verbindung Nr.** | **Dosierung [g a.i./ha]** | **Wirkung gegen Schadpflanzen** | | | |
|---|---|---|---|---|---|
| | | **DIGSA** | **ECHCG** | **LOLMU** | **SETVI** |
| 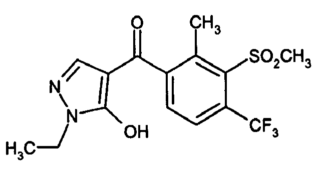 | 80 | 100% | 90% | 60% | 100% |
| erfindungsgemäße Verbindung | | | | | |
| 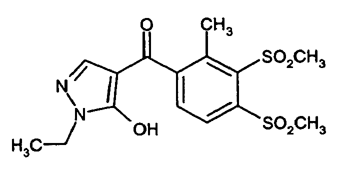 | 80 | 80% | 80% | 20% | 80% |
| aus dem StdT bekannte Verbindung | | | | | |

### Vergleichstabelle 11: Nachauflauf

| **Verbindung Nr.** | **Dosierung [g a.i./ha]** | **Wirkung gegen Schadpflanzen** | | | |
|---|---|---|---|---|---|
| | | **MATIN** | **VERPE** | **VIOTR** | **XANST** |
| 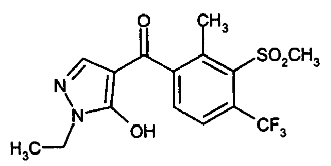 | 80 | 90% | 90% | 80% | 100% |
| erfindungsgemäße Verbindung | | | | | |
| 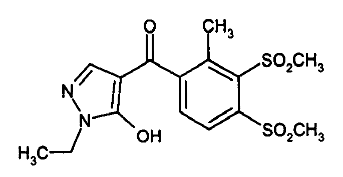 | 80 | 40% | 80% | 50% | 70% |
| aus dem StdT bekannte Verbindung | | | | | |

## Patentansprüche

1. 4-(4-Trifluormethyl-3-thiobenzoyl)pyrazole der Formel (I) oder deren Salze 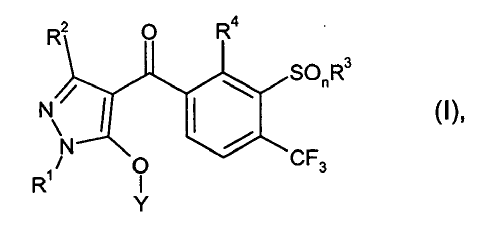 worin
R¹ bedeutet (C₁-C₄)-Alkyl,
R² bedeutet Wasserstoff oder (C₁-C₄)-Alkyl,
R³ bedeutet (C₁-C₄)-Alkyl,
R⁴ bedeutet (C₁-C₄)-Alkyl,
Y bedeutet Wasserstoff, (C₁-C₆)-Alkylsulfonyl, (C₁-C₄)-Alkoxy-(C₁-C₆)-alkylsulfonyl, oder jeweils durch m gleiche oder verschiedene Reste aus der Gruppe bestehend aus Halogen, (C₁-C₄)-Alkyl und (C₁-C₄)-Alkoxy substituiertes Phenylsulfonyl, Thiophenyl-2-sulfonyl, Benzoyl, Benzoyl-(C₁-C₆)-alkyl oder Benzyl,
m bedeutet 0,1, 2 oder 3,
n bedeutet 0, 1 oder 2.

2. 4-(4-Trifluormethyl-3-thiobenzoyl)pyrazole der Formel (I) nach Anspruch 1, worin
R¹ bedeutet Methyl oder Ethyl,
R² bedeutet Wasserstoff, Methyl oder Ethyl,
R³ bedeutet Methyl oder Ethyl,
R⁴ bedeutet Methyl oder Ethyl,
Y bedeutet Wasserstoff, (C₁-C₃)-Alkylsulfonyl, (C₁-C₂)-Alkoxy-(C₁-C₄)-alkylsulfonyl, oder jeweils durch m Methylgruppen substituiertes Phenylsulfonyl, Thiophenyl-2-sulfonyl, Benzoyl, Benzoyl-(C₁-C₆)-alkyl oder Benzyl,
m bedeutet 0 oder 1,
n bedeutet 0, 1 oder 2.

3. 4-(4-Trifluormethyl-3-thiobenzoyl)pyrazole der Formel (I) nach Anspruch 1, worin
R¹ bedeutet Methyl oder Ethyl,
R² bedeutet Wasserstoff, Methyl oder Ethyl,
R³ bedeutet Methyl oder Ethyl,
R⁴ bedeutet Methyl oder Ethyl,
Y bedeutet Wasserstoff,
n bedeutet 0,1 oder 2.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formullerungshilfsmitteln.

6. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach Anspruch 4 oder 5 auf die Pflanzen oder auf den Ort des unerwünschten Pflanzenwachstums appliziert.

7. Verwendung von Verbindungen der Formel (I) gemäß einem der Ansprüche 1 bis 3 oder von herbiziden Mitteln nach Anspruch 4 oder 5 zur Bekämpfung unerwünschter Pflanzen.

8. Verwendung nach Anspruch7, **dadurch gekennzeichnet, daß** die Verbindungen der Formel (I) zur Bekämpfung unerwünschter Pflanzen in Kulturen von Nutzpflanzen eingesetzt werden.

9. Verwendung nach Anspruch 8, **dadurch gekennzeichnet, daß** die Nutzpflanzen transgene Nutzpflanzen sind.

10. Verbindungen der Formel (III) 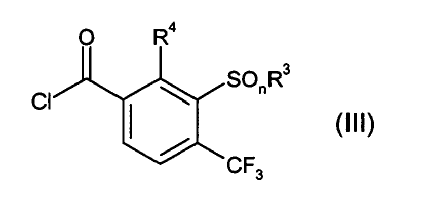 worin R³, R⁴ und n wie in einem der Ansprüche 1 bis 3 definiert sind.

11. Verbindungen der Formel (IIIb) 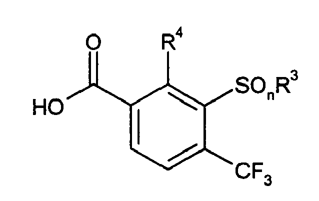 worin R³, R⁴ und n wie in einem der Ansprüche 1 bis 3 definiert sind.
